# EUROPÄISCHE PATENTANMELDUNG

(11) **EP 0 728 481 A2**
(43) Veröffentlichungstag der Anmeldung: **28.08.1996**
(21) Anmeldenummer: 96102129.2
(22) Anmeldetag: 14.02.1996
(51) Int. Cl.: A61K 31/495, A61K 45/06

(54) **Verwendun von Chinoxalinen in Kombination mit Protease-Inhibitoren als Arzneimittel zur Behandlung von AIDS und/oder HIV-Infektionen**

(30) Priorität: 27.02.1995 DE 19506742
(71) Anmelder: BAYER AG, 51368 Leverkusen (DE)
(72) Erfinder: Paessens, Arnold, Dr., D-42781 Haan (DE); Blunck, Martin, Dr., D-42113 Wuppertal (DE); Riess, Günther, Dr., D-65795 Hattersheim (DE); Kleim, Jörg-Peter, Dr., D-65779 Kelkheim (DE); Rösner, Manfred, Dr., D-65817 Eppstein (DE)

(57) **Zusammenfassung**

Die vorliegende Erfindung betrifft die Verwendung von Chinoxalinen in Kombination mit Protease-Inhibitoren als Arzneimittel zur Behandlung von AIDS und/oder HIV-Infektionen.

## Beschreibung

Die vorliegende Erfindung betrifft die Verwendung von Chinoxalinen in Kombination mit Protease-Inhibitoren als Arzneimittel zur Behandlung von AIDS und/oder HIV-Infektionen.

Das Virus der humanen Immundefizienz (HIV) verursacht eine persistent-progrediente, chronische Erkrankung. HIV zerstört das Immunsystem (erworbene Immunschwäche Syndrom, AIDS) und das zentrale und periphere Nervensystem. Daneben werden vielfältige andere klinische Manifestationen im ARC / AIDS-Krankheitsbild durch das HI-Virus mitverursacht - insbesondere opportunistische Infektionen (O.I.), hervorgerufen durch andere Viren, wie z.B. Herpesviren (HSV I und II), Cytomegalovirus (CMV) oder O.I., hervorgerufen durch Bakterien, Pilze oder Parasiten.

HIV gehört zur Familie der Retroviren; eine der wesentlichen und im Vermehrungszyklus essentiellen enzymatischen Aktivitäten dieser Viren ist die Protease (Huff, J.R:, J. Med. Chem. (1991), 34, 2305-2314). Kleinmolekulare peptidische und nicht peptidische Analoga der natürlichen Substrate der Protease hemmen die Replikation des HIV (Roberts, N.A. et al., Science (1990) 248, 358 - 361; Lam, P.Y.S. et al., Science (1994), 263, 380-384).

Analoga der natürlichen Substrate der Reversen Transkriptase wie z.B. Azidothymidin (AZT), Dideoxycytidin (DDC), Dideoxyinosin (DDI) und 3'-Thiacytidin (Lamivudine) hemmen die Replikation des HIV in vitro und in vivo. AZT dient z.B. zur Behandlung von ARC / AIDS-Kranken. Die Langzeittherapie von HIV infizierten Patienten mit AZT geht jedoch mit einer Knochenmarkstoxizität einher; darüberhinaus entstehen AZT-resistente Virusisolate. Unverträglichkeiten wie z.B. eine periphere Neuropathie wird von einigen Patienten berichtet, die mit DDC oder DDI behandelt wurden. Neue Hemmstoffe für die verträgliche und wirksame Therapie sind daher erforderlich.

Die nunmehr aufgefundene Kombination von Chinoxalinen mit Protease-Inhibitoren ist neu und ihre synergistische Wirkung auf die Vermehrung des HIV beim Einsatz in der Bekämpfung von AIDS oder HIV-Infektionen wesentlich besser als der Stand der Technik.

Es wurde jetzt gefunden, daß Chinoxaline der allgemeinen Formeln (I) und (Ia) 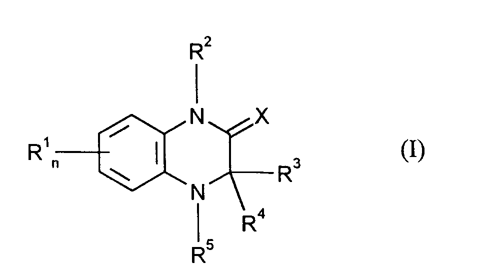 sowie deren tautomere Formen der allgemeinen Formel Ia 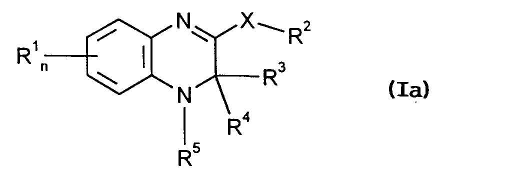 in welchen
1)
   - n: null,
   eins,
   zwei,
   drei,
   oder vier,
die einzelnen Substituenten R¹ unabhängig voneinander Fluor, Chlor, Brom, Jod, Trifluormethyl, Trifluormethoxy, Hydroxy, C₁-C₈-Alkyl, C₅-C₈-Cycloalkyl, C₁-C₆-Alkoxy, (C₁-C₆-Alkoxy)-(C₁-C₄-alkoxy), C₁-C₆-Alkylthio, C₁-C₆-Alkylsulfinyl, C₁-C₆-Alkylsulfonyl, Nitro, Amino, Azido, C₁-C₆-Alkylamino, Di(C₁-C₆-alkyl)amino, Piperidino, Morpholino, 1-Pyrrolidinyl, 4-Methylpiperazinyl, Thiomorpholino, Imidazolyl, Triazolyl, Tetrazolyl, C₁-C₆-Acyl, C₁-C₆-Acyloxy, C₁-C₆-Acylamino, Cyano, Carbamoyl, Carboxy, (C₁-C₆-Alkyl)-oxycarbonyl, Hydroxysulfonyl, Sulfamoyl
oder einen mit bis zu fünf voneinander unabhängigen Resten R⁶ substituierten Phenyl-, Phenoxy-, Phenoxycarbonyl, Phenylthio-, Phenylsulfinyl, Phenylsulfonyl-, Phenoxysulfonyl-, Phenylsulfonyloxy-, Anilinosulfonyl, Phenylsulfonylamino, Benzoyl-, 2-Pyridyl-, 3-Pyridyl- oder 4-Pyridylrest,
wobei R⁶ Fluor, Chlor, Brom, Jod, Cyano, Trifluormethyl, Trifluormethoxy, Nitro, Amino, Azido, C₁-C₆-Alkyl, C₃-C₈-Cycloalkyl, C₁-C₆-Alkoxy, C₁-C₆-Alkylthio, C₁-C₆-Alkylsulfinyl, C₁-C₆-Alkylsulfonyl, C₁-C₆-Alkylamino, Di(C₁-C₆-alkyl)amino, (C₁-C₆-Alkyl)oxycarbonyl, Phenyl, Phenoxy, 2-, 3- oder 4-Pyridyl
sein kann,
- R² und R⁵: gleich oder verschieden, unabhängig voneinander
Wasserstoff, Hydroxy, C₁-C₆-Alkoxy, Aryloxy, C₁-C₆-Acyloxy, Cyano, Amino, C₁-C₆-Alkylamino, Di(C₁-C₆-Alkyl)amino, Arylamino, C₁-C₆-Acylamino, C₁-C₈-Alkyl, gegebenenfalls substituiert mit Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)-amino, C₁-C₆-Alkylthio, C₁-C₆-Alkylsulfonyl, Phenylsulfonyl, Oxo, Thioxo, Carboxy, Carbamoyl;
C₂-C₈-Alkenyl,
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₃-C₈-Allenyl, gegebenenfalls substituiert durch Fluor, Chlor oder Hydroxy, C₁-C₄-alkoxy, Oxo, Phenyl;
C₃-C₈-Alkinyl,
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₃-C₈-Cycloalkyl,
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₃-C₈-Cycloalkenyl,
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
(C₃-C₈-Cycloalkyl)-(C₁-C₄-alkyl),
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
(C₃-C₈-Cycloalkenyl)-(C₁-C₄-alkyl),
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₁-C₆-Alkylcarbonyl
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₂-C₈-Alkenylcarbonyl, gegebenenfalls substituiert durch Fluor, Chlor oder Hydroxy, C₁-C₄-Alkoxy, Oxo, Phenyl;
(C₃-C₈-Cycloalkyl)carbonyl, gegebenenfalls substituiert durch Fluor, Chlor oder Hydroxy, C₁-C₄-Alkoxy, Oxo, Phenyl;
(C₅-C₈-Cycloalkenyl)carbonyl, gegebenenfalls substituiert durch Fluor, Chlor oder Hydroxy, C₁-C₄-Alkoxy, Oxo, Phenyl;
(C₃-C₈-Cycloalkyl)-(C₁-C₃-alkyl)carbonyl, gegebenenfalls substituiert durch Fluor, Chlor oder Hydroxy, C₁-C₄-Alkoxy, Oxo, Phenyl;
(C₅-C₆-Cycloalkenyl)-(C₁-C₃-alkyl)carbonyl, gegebenenfalls substituiert durch Fluor, Chlor oder Hydroxy, C₁-C₄-Alkoxy, Oxo, Phenyl;
C₁-C₈-Alkyloxycarbonyl, gegebenenfalls substituiert durch Fluor, Chlor, Brom, Hydroxy, C₁-C₄-Alkoxy, C₁-C₄-Alkylamino, Di(C₁-C₄-alkyl)amino, C₁-C₄-Alkylthio;
C₂-C₈-Alkenyloxycarbonyl, gegebenenfalls substituiert durch Fluor, Chlor, Hydroxy, C₁-C₄-Alkoxy, Oxo, Phenyl;
C₂-C₈-Alkinyloxycarbonyl, gegebenenfalls substituiert durch Fluor, Chlor, Hydroxy, C₁-C₄-Alkoxy, Oxo, Phenyl;
C₁-C₈-Alkylthiocarbonyl,gegebenenfalls substituiert durch Fluor, Chlor, Hydroxy, C₁-C₄-Alkoxy, Oxo, Phenyl;
C₂-C₈-Alkenylthiocarbonyl, gegebenenfalls substituiert durch Fluor, Chlor, Hydroxy, C₁-C₄-Alkoxy, Oxo, Phenyl;
C₁-C₈-Alkylamino- und Di(C₁-C₈-alkyl)aminocarbonyl, gegebenenfalls substituiert durch Fluor, Chlor, Hydroxy, C₁-C₄-Alkoxy, Oxo, Phenyl;
Pyrrolidin-1-yl, Morpholino-, Piperidino-, Piperazinyl-, oder 4-Methylpiperazin-1-yl-carbonyl, gegebenenfalls substituiert durch C₁-C₄-Alkyl, C₂-C₆-Alkenyl, C₁-C₄-Acyl, Oxo, Thioxo, Carboxy, oder Phenyl;
C₂-C₈-Alkenylamino- und Di(C₁-C₆-alkenyl)aminocarbonyl, gegebenenfalls substituiert durch Fluor, Chlor, Hydroxy, C₁-C₄-Alkoxy, Oxo, Phenyl;
C₁-C₆-Alkylsulfonyl, gegebenenfalls substituiert durch Fluor, Chlor, Hydroxy, C₁-C₄-Alkoxy, Oxo, Phenyl;
C₁-C₆-Alkenylsulfonyl, gegebenenfalls substituiert durch Fluor, Chlor, Hydroxy, C₁-C₄-Alkoxy, Oxo, Phenyl;
oder mit bis zu fünf voneinander unabhängigen Resten R⁶ substituiertes Aryl, Arylcarbonyl, Aryl(thiocarbonyl), (Arylthio)carbonyl, (Arylthio)thiocarbonyl, Aryloxycarbonyl, Arylaminocarbonyl, (Arylamino)thiocarbonyl, Arylalkylaminocarbonyl, Arylsulfonyl, Arylalkyl, Arylalkenyl, Arylalkinyl, Arylalkylcarbonyl, Arylalkenylcarbonyl, Arylalkoxycarbonyl, Aryl(alkylthio)carbonyl, wobei der Alkylrest jeweils 1 bis 5 C-Atome enthalten kann und R⁶ wie oben definiert ist
oder mit bis zu drei voneinander unabhängigen Resten R⁶ substituiertes Heteroaryl, Heteroarylalkyl, Heteroarylalkenyl, Heteroarylalkylcarbonyl oder Heteroarylalkenylcarbonyl, Heteroaryloxycarbonyl, (Heteroarylthio)carbonyl, Heteroarylaminocarbonyl, Heteroarylalkyloxycarbonyl, Heteroaryl(alkylthio)carbonyl, Heteroarylalkylaminocarbonyl, wobei der Alkylrest jeweils 1 bis 3 C-Atome enthalten kann,
- R³ und R⁴: gleich oder verschieden, unabhängig voneinander
Wasserstoff, C₁-C₈-Alkyl, gegebenenfalls substituiert mit Fluor, Chlor, Hydroxy, Amino, Mercapto, C1 - C4-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₂-C₈-Alkenyl, gegebenenfalls substituiert mit Fluor oder Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₃-C₈-Cycloalkyl, gegebenenfalls substituiert mit Fluor, Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₃-C₈-Cycloalkenyl, gegebenenfalls substituiert mit Fluor oder Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
mit bis zu fünf voneinander unabhängigen Resten R⁶ substituiertes Aryl, Arylalkyl, Heteroaryl oder Heteroarylalkyl bedeuten, wobei der Alkylrest jeweils 1 bis 3 C-Atome enthalten kann und R⁶ wie oben definiert ist,
R³ und R⁴ oder R³ und R⁵ können ferner auch
Teil eines gesättigten oder ungesättigten carbo- oder heterocyclischen Ringes mit 3 bis 8 C-Atomen sein, der gegebenenfalls mit Fluor, Chlor, Hydroxy, Amino, C₁-C₆-Alkyl, C₂-C₆-Alkenyl, C₂-C₆-Alkinyl, C₁-C₆-Acyloxy, Benzoyloxy, C₁-C₆-Alkoxy, Oxo, Thioxo, Carboxy, Carbamoyl oder Phenyl substituiert sein kann,
- X: bedeutet Sauerstoff, Schwefel, Selen oder substituierten Stickstoff N-R², worin R² die oben gegebenen Bedeutungen haben kann,
mit Ausnahme der Verbindungen, in denen R³ und R⁴ gleichzeitig H bedeuten und Verbindungen, in denen R² und R⁵ H bedeuten und R³ und/oder R⁴ Arylalkyl bedeuten und Verbindungen, in denen X Sauerstoff und R² und R⁵ Wasserstoff bedeuten,
in Kombination mit Proteaseinhibitoren zur Verwendung als Arzneimittel bei der Bekämpfung von AIDS und HIV-Infektionen sehr gut geeignet sind.

Die in den vorangegangenen Definitionen genannten Alkylgruppen können geradkettig oder verzweigt sein. Sofern nicht anders definiert, enthalten sie vorzugsweise 1-8, besonders bevorzugt 1-6, insbesondere 1-4 C-Atome. Beispiele sind die Methyl-, Ethyl-, Propyl-, 1-Methylethyl-, Butyl-, 1-Methylpropyl-, 2-Methylpropyl-, 1,1-Dimethylethylgruppe und ähnliche.

Die in den vorangegangenen Definitionen genannten Alkenylgruppen können geradkettig oder verzweigt sein und enthalten 1 bis 3 Doppelbindungen. Sofern nicht anders definiert, enthalten diese Gruppen vorzugsweise 2-8, insbesondere 2-6 C-Atome. Beispiele sind die 2-Propenyl-, 1-Methylethenyl, 2-Butenyl-, 3-Butenyl-, 2-Methyl-2-propenyl-, 3-Methyl-2-butenyl, 2,3-Dimethyl-2-butenyl, 3,3-Dichlor-2-propenyl, Pentadienylgruppe und ähnliche.

Die in den vorangegangenen Definitionen genannten Alkinylgruppen können geradkettig oder verzweigt sein und enthalten 1 bis 3 Dreifachbindungen. Sofern nicht anders definiert, enthalten sie vorzugsweise 2-8, besonders bevorzugt 3-6 C-Atome. Beispiele sind die 2-Propinyl- und 3-Butinylgruppe und ähnliche.

Die in den vorangegangenen Definitionen genannten Cycloalkyl- und Cycloalkenylgruppen enthalten, sofern nicht anders definiert, vorzugsweise 3-8, besonders bevorzugt 4-6 C-Atome. Beispiele sind die Cyclopropyl-, Cyclobutyl-, Cyclopentyl-, Cyclopentenyl-, Cyclohexyl- oder Cyclohexenylgruppe.

Die in den vorangegangenen Definitionen genannten Acylgruppen können aliphatisch, cycloaliphatisch oder aromatisch sein. Sofern nicht anders definiert, enthalten sie vorzugsweise 1-8, besonders bevorzugt 2-7 C-Atome. Beispielhafte Acylgruppen sind die Formyl-, Acetyl-, Chloracetyl-, Trifluoracetyl-, Hydroxyacetyl-, Propionyl-, Butyryl-, Isobutyryl-, Pivaloyl-, Cyclohexanoyl- oder Benzoylgruppe.

Die in den vorausgegangenen Definitionen genannten Arylgruppen sind vorzugsweise aromatische Gruppen mit 6-14 C-Atomen, insbesondere mit 6-10 C-Atomen wie z.B. Phenyl, Naphthyl.

In den obengenannten heterocyclischen Ringen bzw. Heteroarylgruppen kommen als Heteroatome insbesondere zum Beispiel O, S, N in Betracht, wobei im Falle eines an dieser Stelle gesättigten N-haltigen Ringes N-Z vorliegt, worin Z, H oder R⁵ mit den jeweiligen oben beschriebenen Definitonen bedeutet.

Soweit nicht anders definiert, haben die heterocyclischen Ringe vorzugsweise 1-13 C-Atome und 1-6 Heteroatome, insbesondere 3-9 C-Atome und 1-4 Heteroatome.

Für die in den vorangegangenen Definitionen genannten Heteroarylgruppen kommen beispielsweise heteroaromatische Reste wie 2- oder 3- Thienyl, 2- oder 3-Furyl, 2-, 3- oder 4- Pyridyl, Pyrimidyl, Indolyl, Chinolyl oder Isochinolyl in Frage.

Die in den vorausgegangenen Definitionen aufgeführten Aralkylgruppen sind beispielsweise Benzyl, Phenylethyl, Naphthylmethyl oder Styryl.

Die obengenannten Substituenten R¹ bis R⁵ sind vorzugsweise 3-fach, besonders bevorzugt 2-fach, insbesondere einfach mit den jeweils angegebenen Substituenten substituiert.

Für die jeweiligen zusammengesetzten Substituentendefinitionen (wie z.B. Arylalkoxycarbonyl) sind die zuvor als bevorzugt beschriebenen Bereiche für die einzelnen Substituenten ebenfalls bevorzugt.

In Abhängigkeit von den verschiedenen Substituenten können Verbindungen der Formeln I und Ia mehrere asymmetrische Kohlenstoffatome besitzen.

Gegenstand der Erfindung sind deshalb sowohl die reinen Stereoisomeren als auch Mischungen derselben, wie z.B. das zugehörige Racemat.

Die reinen Stereoisomeren der Verbindungen der Formeln I und Ia lassen sich durch bekannte Methoden oder in Analogie zu bekannten Methoden direkt herstellen oder nachträglich trennen.

Im Rahmen der Erfindung stehen Proteaseinhibitoren für bekannte strukturunterschiedliche Peptidanaloga, die zur Behandlung von Retrovirus-induzierten Erkrankungen geeignet sind.

Insbesondere seien genannt:
1. (S)-N-[(alphaS)-alpha-[(1R)-2-[((3S,4aS,8aS)-3-(tert.butylcarbamoyl)octahydro-2(1H)-isoquinolinyl)-1-hydroxyethyl)phenethyl-2-quinaldamido]succinamid (EP 432 695 A2) 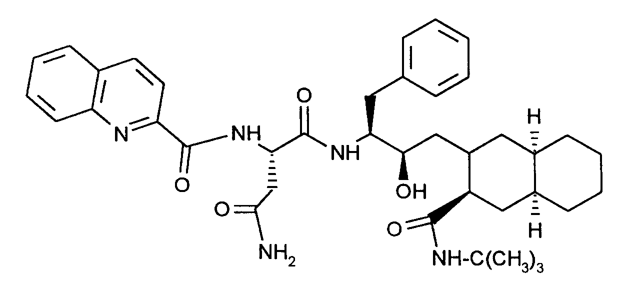
2. 2(R)-Benzyl-5-(2(S)-(N-tert.butylcarbamoyl)-4-(3-pyridylmethyl)piperazin-1-yl)-4(S)-hydroxy-N-(2(R)-hydroxyindan-1(S)-yl)pentanamide (L-735524, EP 569 083 A1, EP 541 168 A1) 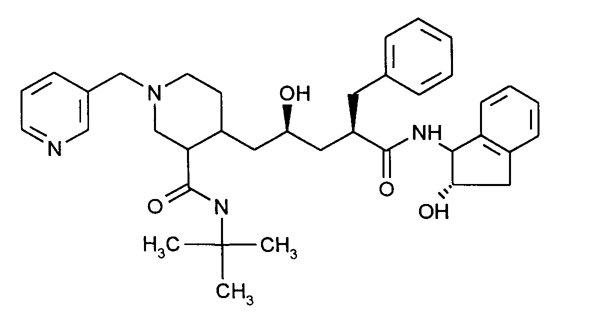
3. N-(Quinolin-2-ylcarbonyl)-asparagine-1(S)-benzyl-3-(3-tert.butyl-1-isobutylureido)-2(R)-hydroxypropylamide (SC 52 151, PCT WO 92/08688 A1, WO 92/08699 A1, WO 92/08698 A1, WO 92/08701 A1, WO 92/08700 A1) 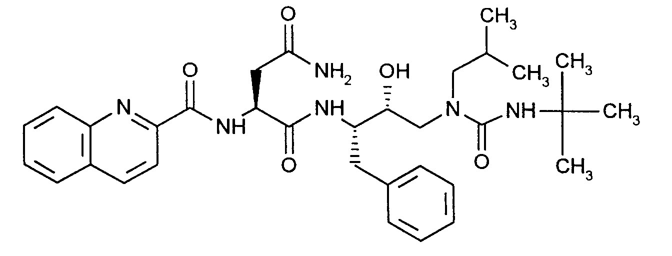
4. N1-(2R-hydroxy-3-((3-methylbutyl)methylsulfonyl)amino)-1S-(phenylmethyl)propyl)-2S-((2-quinolinylcarbonyl)amino)butanediamid (AM 11 686, PCT WO 94/04492) 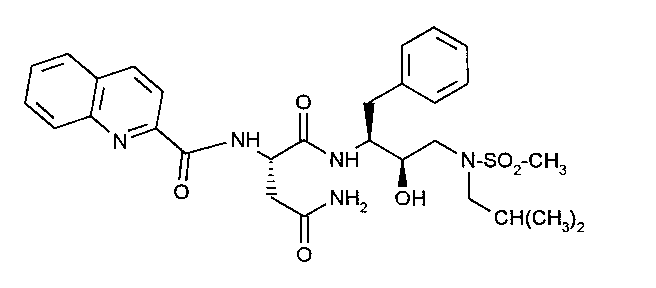
5. (2S,3S,5S)-5(N-(N-((N-methyl-N-((2-isopropyl-4-oxazolyl)methyl)amino)carbonyl)valinyl)amino)-2-(N-((5-thiazolyl)methoxycarbonyl)amino)-1,6-diphenyl-3-hydroxyhexane (A 84 538, PCT WO 94/14436) 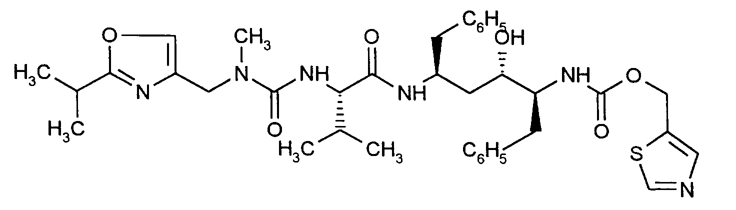
6. (R)-N-tert.butyl-3-((2S,3S)-2-hydroxy-3-N-((R)-2-N-(isoquinolin-5-yloxyacetyl)amino-3-methylthiopropanoyl)amino-4-phenylbutanoyl)-5,5-dimethyl-1,3-thiazolidine-4-carboxamide (KNI 272 / Nippon Mining) 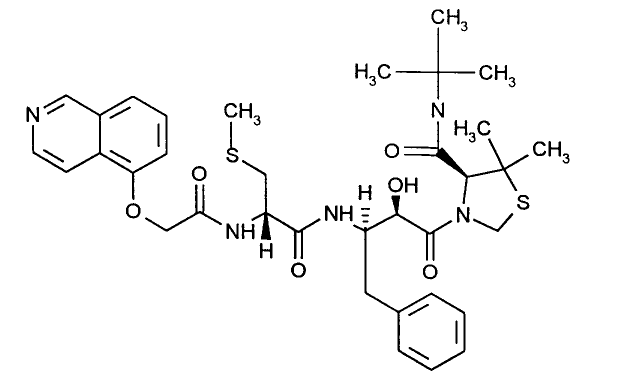
7. {3-[(4-Amino-benzenesulfonyl)-isobutyl-amino]-1-benzyl-2-hydroxypropyl}-carbamic acid tetrahydro-furan-3-yl-ester 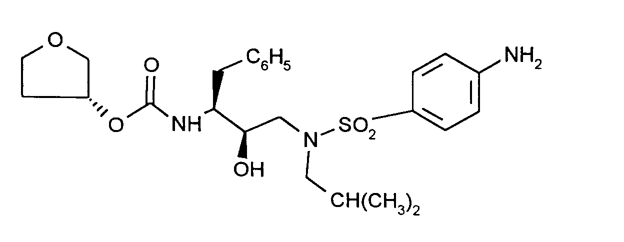
8. (3S,6R)-3-(α-ethylbenzyl)-6-(α-ethylphenethyl)-4-hydroxy-2H-pyran-2-one (VB 11 478, PCT WO 9411361) 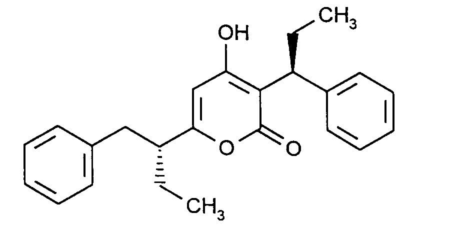
9. N-[5-L-[N-(2-quinolinecarbonyl)-L-asparaginyl]amino-(4R,3S)-epoxy-6-phenyl-hexanoyl]-isoleucin (EP 601 486 A) 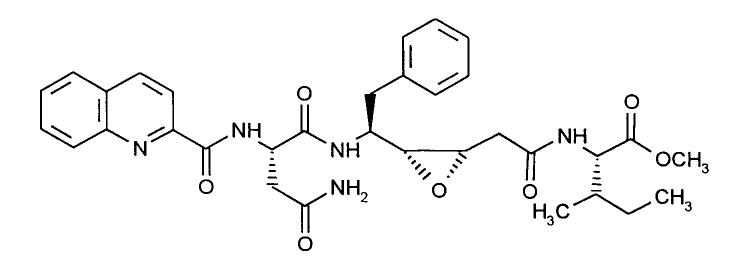
10. N-tert.butyl-1-[2-(R)-hydroxy-4-phenyl)-3-(S)-[[N-(2-quinolinylcarbonyl)asparaginyl]amino]butyl-4(R)-(phenylthio)piperidin-2(S)-carboxamide (EP 560 268 A) 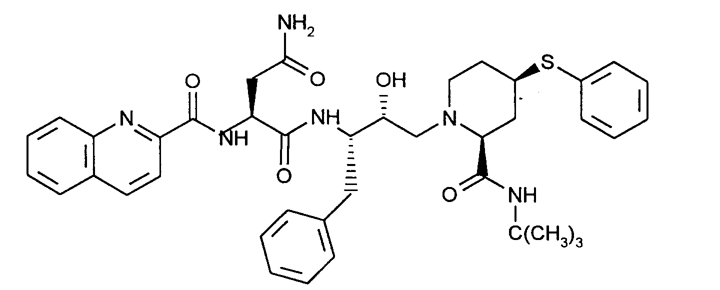
11. [3'''S-(3'''R*,4'''S*)]-N-[1'-oxo-1'-(3''-[1'''-oxo-2'''-aza-3'''-phenylmethyl-4'''-hydroxy-5'''-(2'''-N-tert.butylcarbamido)phenyl]pentyl-4''-methyl)-1,2,3,4-tetrahydroisoquinoline (EP 609 625 A) 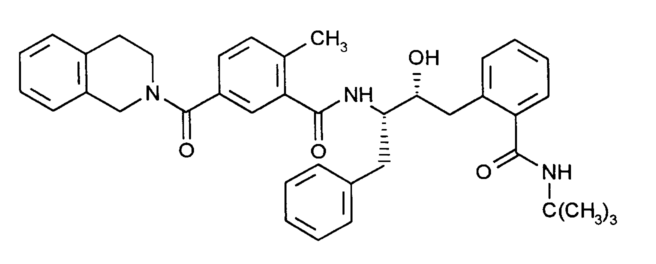
12. 2-[2-Hydroxy-3-(3-hydroxy-2-methyl-benzoylamino)-4-phenylsulfanylbutyl]-decahydro-isoquinoline-3-carboxylicacid-tert.butylamide (AG 1343 Agouron Pharmaceuticals Inc., San Diego USA) 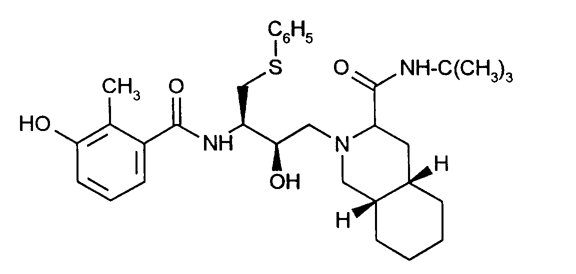
13. 2H-1,4-Diazepin-2-one,hexahydro-6-hydroxy-1,3,4,7-tetrakis(phenylmethyl)-, [3S'-(3.alpha., 6.beta., 7.beta,)] (PCT WO 94/08977)

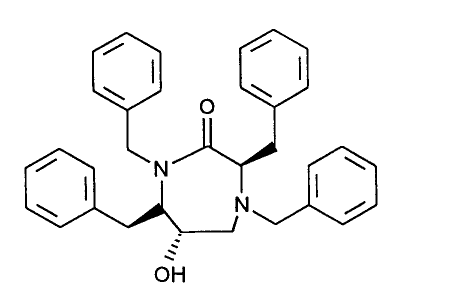

Bevorzugt werden Chinoxaline der allgemeinen Formeln (I) und (Ia),
in welchen
2)
   - n: null,
   eins,
   zwei
   oder drei,
die einzelnen Substituenten R¹ unabhängig voneinander
Fluor, Chlor, Brom, Trifluormethyl, Trifluormethoxy, Hydroxy, C₁-C₄-Alkyl, C₅-C₆-Cycloalkyl, C₁-C₄-Alkoxy, (C₁-C₄-Alkoxy)-(C₁-C₄-alkoxy), C₁-C₄-Alkylthio, C₁-C₄-Alkyl-sulfinyl, C₁-C₄-Alkylsulfonyl, Nitro, Amino, C₁-C₄-Alkylamino, Di-(C₁-C₄-alkyl)amino, Piperidino, Morpholino, 1-Pyrrolidinyl, 4-Methyl-piperazinyl, Thiomorpholino, Imidazolyl, C₁-C₄-Acyl, C₁-C₄-Acyloxy, C₁-C₄-Acylamino, Cyano, Carbamoyl, Carboxy, (C₁-C₄-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenoxycarbonyl, Phenylthio-, Phenylsulfinyl, Phenylsulfonyl-, Phenoxysulfonyl-, Phenylsulfonyloxy-, Anilinosulfonyl, Phenylsulfonylamino, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C₁-C₄-Alkyl, C₃-C₇-Cycloalkyl, C₁-C₄-Alkoxy, C₁-C₄-Alkylthio, C₁-C₄-Alkyl-sulfinyl, C₁-C₄-Alkylsulfonyl, C₁-C₄-Alkylamino, Di(C₁-C₄-alkyl)-amino, (C₁-C₄-Alkyl)-oxycarbonyl, Phenyl, Phenoxy
sein kann,
R² Wasserstoff und R⁵
Wasserstoff, Hydroxy, Cyano, Amino,
C₁-C₆-Alkyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₂-C₈-Alkenyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₃-C₈-Allenyl,
C₃-C₈-Alkinyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₃-C₈-Cycloalkyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₃-C₈-Cycloalkenyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
(C₃-C₈-Cycloalkyl)-(C₁-C₂-alkyl),
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
(C₃-C₈-Cycloalkenyl)-(C₁-C₂-alkyl),
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₁-C₆-Alkylcarbonyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₂-C₆-Alkenylcarbonyl, gegebenenfalls substituiert durch Fluor, Chlor oder Hydroxy, C₁-C₄-alkoxy, Oxo, Phenyl;
(C₃-C₆-Cycloalkyl)carbonyl, gegebenenfalls substituiert durch Fluor, Chlor oder Hydroxy, C₁-C₄-alkoxy, Oxo, Phenyl;
(C₅-C₆-Cycloalkenyl)carbonyl, gegebenenfalls substituiert durch Fluor, Chlor oder Hydroxy, C₁-C₄-alkoxy, Oxo, Phenyl;
(C₃-C₆-Cycloalkyl)-(C₁-C₂-alkyl)carbonyl, gegebenenfalls substituiert durch Fluor, Chlor oder Hydroxy, C₁-C₄-alkoxy, Oxo, Phenyl;
(C₅-C₆-Cycloalkenyl)-(C₁-C₂-alkyl)carbonyl, gegebenenfalls substituiert durch Fluor, Chlor oder Hydroxy, C₁-C₄-alkoxy, Oxo, Phenyl;
C₁-C₆-Alkyloxycarbonyl, gegebenenfalls substituiert durch Fluor, Chlor, Brom, Hydroxy, C₁-C₄-Alkoxy, C₁-C₄-Alkylamino, Di(C₁-C₄-alkyl)amino, C₁-C₄-Alkylthio;
C₂-C₆-Alkenyloxycarbonyl, gegebenenfalls substituiert durch Fluor, Chlor, Hydroxy, C₁-C₄-Alkoxy, Oxo, Phenyl;
C₂-C₆-Alkinyloxycarbonyl, gegebenenfalls substituiert durch Fluor, Chlor, Hydroxy, C₁-C₄-Alkoxy, Oxo, Phenyl;
C₁-C₆-Alkylthiocarbonyl, gegebenenfalls substituiert durch Fluor, Chlor, Hydroxy, C₁-C₄-Alkoxy, Oxo, Phenyl;
C₂-C₆-Alkenylthiocarbonyl, gegebenenfalls substituiert durch Fluor, Chlor, Hydroxy, C₁-C₄-Alkoxy, Oxo, Phenyl;
C₁-C₆-Alkylamino- und Di(C₁-C₆-alkyl)aminocarbonyl, gegebenenfalls substituiert durch Fluor, Chlor, Hydroxy, C₁-C₄-Alkoxy, Oxo, Phenyl;
Pyrrolidin-1-yl, Morpholino-, Piperidino-, Piperazinyl-, oder 4-Methylpiperazin-1-yl-carbonyl;
C₂-C₆-Alkenylamino- und Di(C₁-C₆-alkenyl)aminocarbonyl, gegebenenfalls substituiert durch Fluor, Chlor, Hydroxy, C₁-C₄-Alkoxy, Oxo, Phenyl;
C₁-C₄-Alkylsulfonyl, gegebenenfalls substituiert durch Fluor, Chlor, Hydroxy, C₁-C₄-Alkoxy, Oxo, Phenyl;
C₁-C₄-Alkenylsulfonyl, gegebenenfalls substituiert durch Fluor, Chlor, Hydroxy, C₁-C₄-Alkoxy, Oxo, Phenyl;
oder mit bis zu drei voneinander unabhängigen Resten R⁶ substituiertes Aryl, Arylcarbonyl, Aryl(thiocarbonyl), (Arylthio)carbonyl, (Arylthio)thiocarbonyl, Aryloxycarbonyl, Arylaminocarbonyl, (Arylamino)thiocarbonyl, Arylalkylaminocarbonyl, Arylsulfonyl, Arylalkyl, Arylalkenyl, Arylalkinyl, Arylalkylcarbonyl, Arylalkenylcarbonyl, Aryl(alkylthio)carbonyl, Arylalkoxycarbonyl, wobei der Alkylrest jeweils 1 bis 5 C-Atome enthalten kann und R⁶ wie oben definiert ist
oder mit bis zu zwei voneinander unabhängigen Resten R⁶ substituiertes 1- oder 2-Naphthylmethyl, 2-, 3- oder 4-Picolyl, 2- oder 3-Furylmethyl, 2- oder 3-Thienylmethyl, 2- oder 3-Pyrrolylmethyl, 2-, 3- oder 4-Pyridylcarbonyl, 2- oder 3-Furylcarbonyl, 2- oder 3-Thienylcarbonyl, 2- oder 3-Thienylacetyl, 2-, 3- oder 4-Picolyloxycarbonyl, 2-, oder 3-Furylmethyloxycarbonyl, 2- oder 3-Thienylmethyloxycarbonyl,
und
- R³ und R⁴: gleich oder verschieden, unabhängig voneinander
Wasserstoff,
C₁-C₆-Alkyl,
gegebenenfalls substituiert mit Fluor, Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₂-C₈-Alkenyl, gegebenenfalls substituiert mit Fluor oder Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₃-C₈-Cycloalkyl, gegebenenfalls substituiert mit Fluor, Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₃-C₈-Cycloalkenyl, gegebenenfalls substituiert mit Fluor oder Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl; mit bis zu drei voneinander unabhängigen Resten R⁶ substituiertes Aryl, Arylalkyl, Heteroaryl oder Heteroarylalkyl bedeuten, wobei der Alkylrest jeweils 1 bis 3 C-Atome enthalten kann und R⁶ wie oben definiert ist,
- R³ und R⁴: können ferner auch
Teil eines gesättigten oder ungesättigten carbo- oder heterocyclischen Ringes mit 3 bis 7 C-Atomen sein, der gegebenenfalls mit Fluor, Chlor, Hydroxy, Amino, C₁-C₄-Alkyl, C₂-C₄-Alkenyl, C₂-C₄-Alkinyl, C₁-C₄-Acyloxy, Benzoyloxy, C₁-C₄-Alkoxy, Oxo, Thioxo, Carboxy, Carbamoyl oder Phenyl substituiert sein kann,
X bedeutet Sauerstoff, Schwefel oder Selen
gegebenenfalls in einer isomeren Form, in Kombination mit Proteaseinhibitoren der Reihe:
1. (S)-N-[(alphaS)-alpha[(1R)-2-[((3S,4aS,8aS)-3-(tert.butylcarbamoyl)octahydro-2(1H)-isoquinolinyl)-1-hydroxyethyl)phenethyl-2-quinaldamido]succinamid
2. 2(R)-Benzyl-5-(2(S)-(N-tert.butylcarbamoyl)-4-(3-pyridylmethyl)piperazin-1-yl)-4(S)-hydroxy-N(2(R)-hydroxyindem-1(S)-yl)pentanamide
3. N-(Quinolin-2-ylcarbonyl)-asparagine-1(S)-benzyl-3-(3-tert.butyl-1-isobutylureido)-2(R)-hydroxypropylamide
4. N1-(2R-hydroxy-3-((3-methylbutyl)methylsulfonyl)amino)-1S-(phenylmethyl)propyl)-2S-((2-quinolinylcarbonyl)amino)butanediamid
5. (2S,3S,5S)-5(N-(N-(N-methyl-N-((2-isopropyl-4-oxazolyl)methyl)amino)carbonyl)valinyl)amino)-2-(N-((5-thiazolyl)methoxycarbonyl)amino)-1,6-diphenyl-3-hydroxyhexane
6. (R)-N-tert.butyl-3-((2S,3S)-2-hydroxy-3-N-((R)-2-N-(isoquinolin-5-yloxyacetyl)amino-3-methylthiopropanoyl)amino-4-phenylbutanoyl)-5,5-dimethyl-1,3-thiazolidine-4-carboxamide
7. {3-[(4-Amino-benzenesulfonyl)-isobutyl-amino]-1-benzyl-2-hydroxypropyl}-carbamic acid tetrahydro-furan-3-yl-ester
8. (3S,6R)-3-(α-ethylbenzyl)-6-(α-ethylphenethyl)-4-hydroxy-2H-pyran-2-one (VB 11 478, PCT WO 9411361)
9. N-[5-L-[N-(2-quinolinecarbonyl)-L-asparaginyl]amino-(4R,3S)-epoxy-6-phenyl-hexanoyl]-isoleucin
10. N-tert.butyl-1-[2-(R)-hydroxy-4-phenyl)-3(S)-[[N-(2-quinolinylcarbonyl)asparaginyl]amino]butyl-4(R)-(phenylthio)piperidin-2(S)-carboxamide
11. [3'''S-(3'''R*,4'''S*)]-N[1'-oxo-1'-(3''-[1'''-oxo-2'''-aza-3'''-phenylmethyl-4'''-hydroxy-5'''-(2'''-N-tert.butylcarbamido)phenyl]pentyl-4''-methyl)-1,2,3,4-tetrahydroisoquinoline
12. 2-[2-Hydroxy-3-(3-hydroxy-2-methyl-benzoylamino)-4-phenylsulfanylbutyl]-decahydro-isoquinoline-3-carboxylicacid-tert.butylamide
13. 2H-1,4-Diazepin-2-one,hexahydro-6-hydroxy-1,3,4,7-tetrakis(phenylmethyl)-, [3S-(3.alpha., 6.beta., 7.beta)]
zur Verwendung als Arzneimittel bei der Behandlung von AIDS- bzw. HIV-Infektionen.

Besonders bevorzugt werden Chinoxaline der allgemeinen Formeln (I) und (Ia),
in welcher
- n: null,
eins
oder zwei,
die einzelnen Substituenten R¹ unabhängig voneinander
Fluor, Chlor, Brom, Trifluormethyl, Hydroxy, C₁-C₄-Alkyl, C₁-C₄-Alkoxy, (C₁-C₄-Alkoxy)-(C₁-C₂-alkoxy), C₁-C₄-Alkylthio, Nitro, Amino, C₁-C₄-Alkylamino, Di(C₁-C₄-alkyl)amino, Piperidino, Morpholino, 1-Pyrrolidinyl, 4-Methylpiperazinyl, C₁-C₄-Acyl, C₁-C₄-Acyloxy, C₁-C₄-Acylamino, Cyano, Carbamoyl, Carboxy, (C₁-C₄-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C₁-C₄-Alkyl, C₁-C₄-Alkoxy, (C1 - C4-Alkyl)-oxycarbonyl, Phenyl, Phenoxy sein kann,
R² Wasserstoff und R⁵
C₁-C₆-Alkyl
gegebenenfalls substituiert mit C₁-C₄-Alkoxy oder C₁-C₄-Alkylthio;
C₂-C₆-Alkenyl,
gegebenenfalls substituiert mit Oxo;
C₃-C₆-Allenyl;
C₃-C₈-Alkinyl, insbesondere 2-Butinyl;
C₃-C₆-Cycloalkyl;
C₅-C₆-Cycloalkenyl;
(C₃-C₆-Cycloalkyl)-(C₁-C₂-alkyl), insbesondere Cyclopropylmethyl, gegebenenfalls substituiert mit C₁-C₄-Alkyl;
(C₃-C₆-Cycloalkenyl)-(C₁-C₂-alkyl), insbesondere Cyclohexenylmethyl;
C₁-C₆-Alkylcarbonyl,
gegebenenfalls substituiert mit Fluor,
Chlor, Hydroxy, Benzyloxy, Phenoxy, C₁-C₄-Alkoxy, C₁-C₄-Alkylamino, C₁-C₄-Alkenylamino, Di(C₁-C₄-alkyl)amino, 1-Pyrrolidinyl, Piperidino, Morpholino, 4-Methylpiperazin-1-yl, C₁-C₄-Alkylthio;
C₂-C₆-Alkenylcarbonyl;
C₁-C₆-Alkyloxycarbonyl, gegebenenfalls substituiert durch Fluor, Chlor, Brom, Hydroxy, C₁-C₄-Alkoxy, C₁-C₄-Alkylamino, Di(C₁-C₄-alkyl)amino, C₁-C₄-Alkylthio;
C₂-C₆-Alkenyloxycarbonyl, insbesondere Vinyloxycarbonyl, Allyloxycarbonyl, Isopropenyloxycarbonyl, Butenyloxycarbonyl, Pentenyloxycarbonyl;
C₂-C₆-Alkinyloxycarbonyl, insbesondere Propinyloxycarbonyl, Butinyloxycarbonyl;
C₁-C₆-Alkylthiocarbonyl;
C₂-C₆-Alkenylthiocarbonyl, insbesondere Allylthiocarbonyl;
C₁-C₆-Alkylamino- und Di(C₁-C₆-alkyl)aminocarbonyl;
Pyrrolidin-1-yl, Morpholino-, Piperidino-, Piperazinyl-, oder 4-Methylpiperazin-1-yl-carbonyl;
C₂-C₆-Alkenylamino- und Di(C₁-C₆-alkenyl)aminocarbonyl;
C₁-C₄-Alkylsulfonyl;
C₁-C₄-Alkenylsulfonyl;
oder mit bis zu zwei voneinander unabhängigen Resten R⁶ substituiertes Aryl, insbesondere Phenyl, Arylcarbonyl, insbesondere Benzoyl, (Arylthio)carbonyl, Aryloxycarbonyl, Arylaminocarbonyl, (Arylamino)thiocarbonyl, Arylalkylaminocarbonyl, Arylsulfonyl, Arylalkyl, insbesondere Benzyl, Phenylethyl, Arylalkenyl, Arylalkylcarbonyl, Arylalkoxycarbonyl, Aryl(alkylthio)carbonyl, wobei der Alkylrest jeweils 1 bis 3 C-Atome enthalten kann und R⁶ wie oben definiert ist
oder mit bis zu zwei voneinander unabhängigen Resten R⁶ substituiertes 1- oder 2-Naphthylmethyl, 2-, 3- oder 4-Picolyl, 2- oder 3-Furylmethyl, 2- oder 3-Thienylmethyl, 2- oder 3-Pyrrolylmethyl, 2-, 3- oder 4-Pyridylcarbonyl, 2- oder 3-Furylcarbonyl, 2- oder 3-Thienylcarbonyl, 2- oder 3-Thienylacetyl, 2-, 3- oder 4-Picolyloxycarbonyl, 2- oder 3-Furylmethyloxycarbonyl, 2- oder 3-Thienylmethyloxycaronyl,
und
- R³ und R⁴: gleich oder verschieden, unabhängig voneinander
Wasserstoff,
C₁-C₄-Alkyl,
gegebenenfalls substituiert mit Hydroxy, Mercapto, C₁-C₄-Alkoxy, C₁-C₄-Alkylthio, C₁-C₄-Alkylsulfonyl, C₁-C₄-Alkylsulfinyl, Carboxy, Carbamoyl;
C₂-C₆-Alkenyl,
mit bis zu zwei voneinander unabhängigen Resten R⁶ substituiertes Aryl, Benzyl, Thienyl oder Thienylmethyl, wobei R⁶ wie oben definiert ist, bedeuten,
R³ und R⁴ können auch
Teil eines gesättigten oder ungesättigten carbo- oder heterocyclischen Ringes mit 3 bis 6 C-Atomen sein, der gegebenenfalls mit Oxo oder Thioxo substituiert sein kann und
- X: bedeutet Sauerstoff oder Schwefel
gegebenenfalls in einer isomeren Form in Kombination mit Proteaseinhibitoren der Reihe:
1. (S)-N-[(alphaS)-alpha-[(1R)-2[((3S,4aS,8aS)-3-(tert.butylcarbamoyl)octahydro-2(1H)-isoquinolinyl)-1-hydroxyethyl)phenethyl-2-quinaldamido]succinamid
2. 2(R)-Benzyl-5-(2(S)-(N-tert.butylcarbamoyl)-4-(3-pyridylmethyl)piperazin-1-yl)-4(S)-hydroxy-N(2(R)-hydroxyindem-1(S)-yl)pentanamide
3. N-(Quinolin-2-ylcarbonyl)-asparagine-1(S)-benzyl-3-(3-tert.butyl-1-isobutylureido)-2(R)-hydroxypropylamide
4. N1-(2R-hydroxy-3-((3-methylbutyl)methylsulfonyl)amino)-1S-(phenylmethyl)propyl)-2S-((2-quinolinylcarbonyl)amino)butanediamid
5. (2S,3S,5S)-5(N-(N-((N-methyl-N-((2-isopropyl-4-oxazolyl)methyl)amino)carbonyl)valinyl)amino)-2-(N-((5-thiazolyl)methoxycarbonyl)amino)-1,6-diphenyl-3-hydroxyhexane
6. (R)-N-tert.butyl-3-((2S,3S)-2-hydroxy-3-N-((R)-2-N-(isoquinolin-5-yloxyacetyl)amino-3-methylthiopropanoyl)amino-4-phenylbutanoyl)-5,5-dimethyl-1,3-thiazolidine-4-carboxamide
7. {3-[(4-Amino-benzenesulfonyl)-isobutyl-amino]-1-benzyl-2-hydroxypropyl}-carbamic acid tetrahydro-furan-3-yl-ester
8. (3S,6R)-3-(α-ethylbenzyl)-6-(α-ethylphenethyl)-4-hydroxy-2H-pyran-2-one (VB 11 478, PCT WO 9411361)
9. N-[5-L-[N-(2-quinolinecarbonyl)-L-asparaginyl]amino-(4R,3S)-epoxy-6-phenyl-hexanoyl]-isoleucin
10. N-tert.butyl-1-[2-(R)-hydroxy-4-phenyl)-3(S)-[[N-(2-quinolinylcarbonyl)asparaginyl]amino]butyl-4(R)-(phenylthio)piperidin-2(S)-carboxamide
11. [3'''S-(3'''R*,4'''S*)]-N[1'-oxo-1'-(3''-[1'''-oxo-2'''-aza-3'''-phenylmethyl-4'''-hydroxy-5'''-(2'''-N-tert.butylcarbamido)phenyl]pentyl-4''-methyl)-1,2,3,4-tetrahydroisoquinoline
12. 2-[2-Hydroxy-3-(3-hydroxy-2-methyl-benzoylamino)-4-phenylsulfanylbutyl]-decahydro-isoquinoline-3-carboxylicacid-tert.butylamide
13. 2H-1,4-Diazepin-2-one,hexahydro-6-hydroxy-1,3,4,7-tetrakis(phenylmethyl)-, [3S-(3.alpha., 6.beta., 7.beta)]
zur Verwendung als Arzneimittel bei der Behandlung von AIDS- bzw. HIV-Infektionen.

Ganz besonders bevorzugt ist die Kombination von S-4-Isopropoxycarbonyl-6-methoxy-3-(methylthio-methyl)-3,4-dihydro-chinoxazolin-2(1H)-thion der Formel (A) 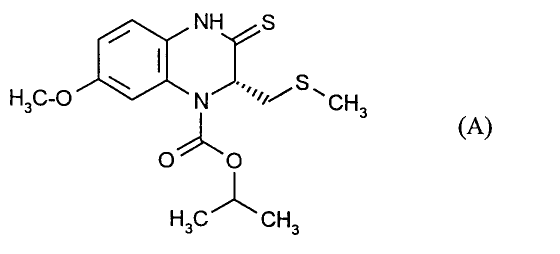 und (S)-N-[(alphaS)-alpha-[(1R)-2-[((3S,4aS,8aS)-3-(tert.butylcarbamoyl)-octahydro-2(1H)-isoquinolinyl)-1-hydroxyethyl)phenethyl-2-quinaldamido]-succinamid (Saquinavir) der Formel (B) 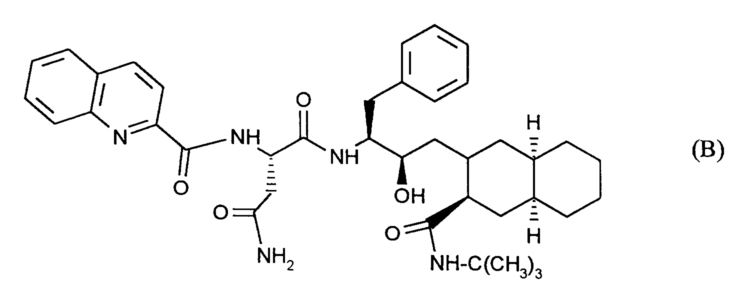 zur Verwendung bei der Bekämpfung von AIDS bzw. HIV-Infektionen.

Die Chinoxaline der allgemeinen Formeln (I) und (Ia) sind bekannt [vgl. EP 509 398 A1]. Ebenso sind die oben aufgeführten Proteaseinhibitoren bekannt [vgl. EP 432 695 A2, EP 569 083 A1, EP 541 168 A1, PCT WO 92/08688 A1, WO 92/08699 A1, WO 92/08698 A1, WO 92/08701 A1, WO 92/08700 A1, PCT WO 94/04492, PCT WO 94/14436, PCT WO 9411361, EP 601 486 A, EP 560 268 A, EP 609 625 A, PCT WO 94/08977].

Die Verwendung der Kombination dieser Verbindungen bietet bei der Behandlung Retrovirus-induzierter - insbesondere aber HIV-induzierter Erkrankungen - Vorteile im Vergleich zur Monotherapie mit den Einzelverbindungen. Der vorteilhafte und überlegene Gebrauch der Kombination dieser Verbindungen zur Behandlung von AIDS- oder HIV-Infektionen ergibt sich hauptsächlich aus der synergistischen antiviralen Wirksamkeit, aber zusätzlich auch aus der unveränderten Verträglichkeit der Substanzen in Kombination im Bereich der Toxizität, bei der 50% der Zellen überleben - im Vergleich zur Tox-50 der Einzelkomponenten. Für andere Kombinationen ist bekannt - z.B. AZT in Kombination mit Ganciclovir, daß bei Verwendung einer Kombination eine synergistische Toxizität erfolgt [vgl. M.N. Prichard et al.; Antimicrob. Agents Chemotherapy (1991), 35, 1060-1065].

Die erniedrigte Wirkdosis, die sich aus dem Gebrauch der Kombination der Substanzen für die Behandlung ergibt, verringert darüberhinaus die Wahrscheinlichkeit der Ausbildung resistenter Virusisolate.

Die Erfindung behandelt die Kombination zweier Verbindungsklassen der HIV Reversen Transkriptase und der HIV-Protease zur Prävention und Behandlung von Infektionen mit dem HIV, sowie zur Behandlung der durch das HIV induzierten Erkrankungen wie AIDS Related Complex (ARC) oder AIDS.

### HIV-Infektion in Zellkultur

Der HIV-Test wurde mit Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phythaemagglutinin (90 µg/ml) und Interleukin-2 (40 U/ml) stimuliert. Zur Infektion mit dem infektiösen HIV wurden PBL's pelletiert, und das Zellpellet wurde anschließend in 1 ml HI-Viruslösung zur Adsorption suspendiert und 1 Stunde bei 37°C inkubiert.

Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96er Mikrotiterplatten pipettiert.
Alternativ wurden anstelle der PBL's H9 Zellen für die antiviralen Tests eingesetzt.

Die Testung der kombinatorischen Wirkung der Prüfsubstanzen wurde mittels Schachbrett-Titration (Chequerboardtitration) durchgeführt.
Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen - alleine oder in entsprechenden Kombinationen - in unterschiedlichen Konzentrationen, ausgehend von den Näpfen der 3. vertikalen Reihe der Mikrotiterplatte, von der die Prüfsubstanzen in 2er Schritten weiter verdünnt wurden (50 µl Volumen pro Napf). Für die Kombination wurden Verdünnungen der 2. Substanz auf einer seperaten 96er Mikrotiterplatte hergestellt und anschließend auf die vorbereitete erste Platte zupipettiert. Dazu wurden jeweils 100 µl der vorbereiteten HIV-infizierten Zellen gegeben (so.). Damit waren Testkonzentrationen im Bereich ca. 10 - 50fach ober- und unterhalb der IC₅₀-Konzentrationen abgedeckt.
Die Testansätze wurden so lange bei 37°C inkubiert, bis in der unbehandelten Viruskontrolle die für das HIV typische Syncytienbildung an der Wirtszelle (zwischen Tag 3 und 6 nach Infektion) mikroskopisch nachweisbar wurde. In der unbehandelten Viruskontrolle resultierten unter diesen Testbedingungen etwa 20 - 50 Syncytien, während die unbehandelte Zellkontrolle keine Syncytien aufwies. Die Überstände der 96er Platte wurden dann geerntet und in einem HIV-spezifischen ELISA-Test auf HIV-spezifisches Antigen untersucht (Vironostika HIV Antigen, Organon Teknika).
Die Hemmwerte wurden entsprechend der Cutt-Off-Werte aus entsprechenden Zell- bzw. Viruskontrollen bzw. interner Testkontrollen in Prozent (%)-Hemmwerte umgerechnet, und die IC₅₀-Werte wurden als die Konzentrationen der behandelten und infizierten Zellen ermittelt, bei der 50% des Virus-spezifischen Antigens durch die Behandlung mit den Verbindungen unterdrückt waren. Zur Analyse der synergistischen Wirksamkeit der Verbindungen wurden die Differenzwerte von errechneten und gemessenen Hemmwerten jeder Kombination ermittelt (Prichard, M.N. et al., Antimicrob. Agents Chemoth. (1993), 37, 540-545).
Differenzwerte > Null bedeuten, daß eine synergistische Wirksamkeit auftritt. Beispielsweise wurden folgende Ergebnisse erzielt:

**Tab. 1**

| Differenztabelle zur berechneten und gemessenen Wirkung von Saquinavir (B) mit Beispiel der Formel (A) | | | | | | | |
|---|---|---|---|---|---|---|---|
| **Beispiel d. Formel (A) nM** | **50** | **25** | **12** | **6** | **3** | **1,5** | **0,7** |
| **Saquinavir (B) nM** | | | | | | | |
| **50** | 0 | 0 | 0 | 13 | 32 | 30 | 16 |
| **25** | 0 | 0 | 0 | 29 | 49 | 20 | 3 |
| **12** | 0 | 0 | 0 | 37 | 40 | 0 | 0 |
| **6** | 0 | 0 | 0 | 32 | 57 | 23 | 0 |
| **3** | 0 | 0 | 0 | 27 | 0 | 0 | 0 |

Im Konzentrationsfenster 0,7 - 6 nM des Beispiels der Formel (A) mit 6 - 50 nM des Proteaseinhibitors (B) resultiert eine synergistische Wirksamkeit der Kombinationen.

Zur Messung einer synergistischen Toxizität der Verbindungen wurden Substanzkonzentrationen um den Tox-50 Wert der Einzelverbindungen geprüft und mikroskopisch auf zelltoxische Eigenschaften untersucht bzw. mittels Trypanblau eine Vitalfärbung durchgeführt. Keine der untersuchten Kombinationen wies eine synergistische Toxizität auf.

Es wurde überraschenderweise gefunden, daß durch die Verwendung der Kombination der Verbindungen eine synergistische Wirkung auf das HIV erzielt wird. Dies wurde beispielhaft durch Kombinationsstudien des Chinoxalinderivates mit Saquinavir (Tab. 1) gezeigt.

Die erfindungsgemäßen Kombinationen dienen der Behandlung und Prophylaxe von Erkrankungen, hervorgerufen durch Retroviren, in der Human- und Tiermedizin.

Als Indikationsgebiete in der Humanmedizin können beispielsweise genannt werden:
1.) Die Behandlung und Prophylaxe von menschlichen Retrovirusinfektionen.
2.) Für die Behandlung oder Prophylaxe von HIV I (Virus der humanen Immundefizienz; früher HTLV III/LAV genannt) verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.
3.) Für die Behandlung oder die Prophylaxe einer HTLV-I oder HTLV-II Infektion.
4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit
a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen) e) infektiösem Virus der Anämie (des Pferdes)
f) Infektionen, verursacht durch das Katzenleukämievirus
g) Infektionen, verursacht durch das Virus der Katzen-Immundefizienz (FIV)
h) Infektionen, verursacht durch das Virus der Affen-Immundefizienz (SIV)

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formeln (I) / (Ia) in Kombination mit einem der angegebenen Proteaseinhibitoren enthalten oder die aus einem oder mehreren Wirkstoffen der Formeln (I) / (Ia) und den Proteaseinhibitoren bestehen, sowie Verfahren zur Herstellung dieser Zubereitungen, insbesondere die Kombination der Prüfverbindungen.

Die Wirkstoffe der Formeln (I) und (Ia) und den Proteaseinhibitoren sollen in den oben aufgeführten pharmazeutischen Zubereitungen, vorzugsweise in einer Konzentration von etwa 0,1 bis 99,5 Gew.-%, vorzugsweise von etwa 0,5 bis 95 Gew.-% der Gesamtmischung, vorhanden sein.

Die oben aufgeführten pharmazeutischen Zubereitungen können außer den Verbindungen der Formeln (I) / (Ia) in Kombination mit einem der oben angegebenen Proteaseinhibitoren auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels, sowie dem Zeitraum bzw. Intervall, innerhalb welchem die Verabreichung erfolgt.

## Patentansprüche

1. Arzneimittel, enthaltend in Kombination einen oder mehrere Proteaseinhibitoren und eins oder mehrere der Chinoxaline der allgemeinen Formel (I) und (Ia) 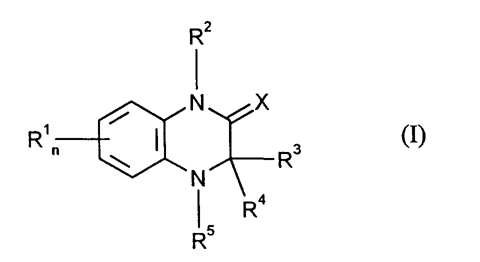 sowie deren tautomere Formen der allgemeinen Formel Ia 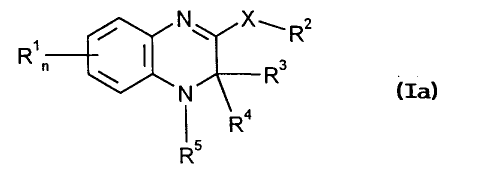 in welchen
1)
n null,
eins,
zwei,
drei,
oder vier,
die einzelnen Substituenten R¹ unabhängig voneinander Fluor, Chlor, Brom, Jod, Trifluormethyl, Trifluormethoxy, Hydroxy, C₁-C₈-Alkyl, C₅-C₈-Cycloalkyl, C₁-C₆-Alkoxy, (C₁-C₆-Alkoxy)-(C₁-C₄-alkoxy), C₁-C₆-Alkylthio, C₁-C₆-Alkylsulfinyl, C₁-C₆-Alkylsulfonyl, Nitro, Amino, Azido, C₁-C₆-Alkylamino, Di(C₁-C₆-alkyl)amino, Piperidino, Morpholino, 1-Pyrrolidinyl, 4-Methylpiperazinyl, Thiomorpholino, Imidazolyl, Triazolyl, Tetrazolyl, C₁-C₆-Acyl, C₁-C₆-Acyloxy, C₁-C₆-Acylamino, Cyano, Carbamoyl, Carboxy, (C₁-C₆-Alkyl)-oxycarbonyl, Hydroxysulfonyl, Sulfamoyl
oder
einen mit bis zu fünf voneinander unabhängigen Resten R⁶ substituierten Phenyl-, Phenoxy-, Phenoxycarbonyl, Phenylthio-, Phenylsulfinyl, Phenylsulfonyl-, Phenoxysulfonyl-, Phenylsulfonyloxy-, Anilinosulfonyl, Phenylsulfonylamino, Benzoyl-, 2-Pyridyl-, 3-Pyridyl- oder 4-Pyridylrest,
wobei R⁶
Fluor, Chlor, Brom, Jod, Cyano, Trifluormethyl, Trifluormethoxy, Nitro, Amino, Azido, C₁-C₆-Alkyl, C₃-C₈-Cycloalkyl, C₁-C₆-Alkoxy, C₁-C₆-Alkylthio, C₁-C₆-Alkylsulfinyl, C₁-C₆-Alkylsulfonyl, C₁-C₆-Alkylamino, Di(C₁-C₆-alkyl)amino, (C₁-C₆-Alkyl)-oxycarbonyl, Phenyl, Phenoxy, 2-, 3- oder 4-Pyridyl
sein kann,
R² und R⁵ gleich oder verschieden, unabhängig voneinander
Wasserstoff, Hydroxy, C₁-C₆-Alkoxy, Aryloxy, C₁-C₆-Acyloxy, Cyano, Amino, C₁-C₆-Alkylamino, Di(C₁-C₆-Alkyl)amino, Arylamino, C₁-C₆-Acylamino, C₁-C₈-Alkyl, gegebenenfalls substituiert mit Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₂-C₈-Alkenyl,
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₃-C₈-Allenyl, gegebenenfalls substituiert durch Fluor, Chlor oder Hydroxy, C₁-C₄-alkoxy, Oxo, Phenyl;
C₃-C₈-Alkinyl,
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₃-C₈-Cycloalkyl,
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₃-C₈-Cycloalkenyl,
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
(C₃-C₈-Cycloalkyl)-(C₁-C₄-alkyl),
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
(C₃-C₈-Cycloalkenyl)-(C₁-C₄-alkyl),
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₁-C₆-Alkylcarbonyl
gegebenenfalls substituiert mit
Fluor, Chlor, Brom, Jod, Cyano, Amino, Mercapto, Hydroxy, C₁-C₆-Acyloxy, Benzoyloxy, Benzyloxy, Phenoxy, C₁-C₆-Alkoxy, C₁-C₆-Alkylamino, Di(C₁-C₆-alkyl)amino, C₁-C₆-Alkylthio, C₁-C₆-Alkylsulfonyl, Phenylsulfonyl, Oxo, Thioxo, Carboxy, Carbamoyl;
C₂-C₈-Alkenylcarbonyl, gegebenenfalls substituiert durch Fluor, Chlor oder Hydroxy, C₁-C₄-Alkoxy, Oxo, Phenyl;
(C₃-C₈-Cycloalkyl)carbonyl, gegebenenfalls substituiert durch Fluor, Chlor oder Hydroxy, C₁-C₄-Alkoxy, Oxo, Phenyl;
(C₅-C₈-Cycloalkenyl)carbonyl, gegebenenfalls substituiert durch Fluor, Chlor oder Hydroxy, C₁-C₄-Alkoxy, Oxo, Phenyl;
(C₃-C₈-Cycloalkyl)-(C₁-C₃-alkyl)carbonyl, gegebenenfalls substituiert durch Fluor, Chlor oder Hydroxy, C₁-C₄-Alkoxy, Oxo, Phenyl;
(C₅-C₆-Cycloalkenyl)-(C₁-C₃-alkyl)carbonyl, gegebenenfalls substituiert durch Fluor, Chlor oder Hydroxy, C₁-C₄-Alkoxy, Oxo, Phenyl;
C₁-C₈-Alkyloxycarbonyl, gegebenenfalls substituiert durch Fluor, Chlor, Brom, Hydroxy, C₁-C₄-Alkoxy, C₁-C₄-Alkylamino, Di(C₁-C₄-alkyl)amino, C₁-C₄-Alkylthio;
C₂-C₈-Alkenyloxycarbonyl, gegebenenfalls substituiert durch Fluor, Chlor, Hydroxy, C₁-C₄-Alkoxy, Oxo, Phenyl;
C₂-C₈-Alkinyloxycarbonyl, gegebenenfalls substituiert durch Fluor, Chlor, Hydroxy, C₁-C₄-Alkoxy, Oxo, Phenyl;
C₁-C₈-Alkylthiocarbonyl, gegebenenfalls substituiert durch Fluor, Chlor, Hydroxy, C₁-C₄-Alkoxy, Oxo, Phenyl;
C₂-C₈-Alkenylthiocarbonyl, gegebenenfalls substituiert durch Fluor, Chlor, Hydroxy, C₁-C₄-Alkoxy, Oxo, Phenyl;
C₁-C₈-Alkylamino- und Di(C₁-C₈-alkyl)aminocarbonyl, gegebenenfalls substituiert durch Fluor, Chlor, Hydroxy, C₁-C₄-Alkoxy, Oxo, Phenyl;
Pyrrolidin-1-yl, Morpholino-, Piperidino-, Piperazinyl-, oder 4-Methylpiperazin-1-yl-carbonyl, gegebenenfalls substituiert durch C₁-C₄-Alkyl, C₂-C₆-Alkenyl, C₁-C₄-Acyl, Oxo, Thioxo, Carboxy, oder Phenyl;
C₂-C₈-Alkenylamino- und Di(C₁-C₆-alkenyl)aminocarbonyl, gegebenenfalls substituiert durch Fluor, Chlor, Hydroxy, C₁-C₄-Alkoxy, Oxo, Phenyl;
C₁-C₆-Alkylsulfonyl, gegebenenfalls substituiert durch Fluor, Chlor, Hydroxy, C₁-C₄-Alkoxy, Oxo, Phenyl;
C₁-C₆-Alkenylsulfonyl, gegebenenfalls substituiert durch Fluor, Chlor, Hydroxy, C₁-C₄-Alkoxy, Oxo, Phenyl;
oder mit bis zu fünf voneinander unabhängigen Resten R⁶ substituiertes Aryl, Arylcarbonyl, Aryl(thiocarbonyl), (Arylthio)carbonyl, (Arylthio)thiocarbonyl, Aryloxycarbonyl, Arylaminocarbonyl, (Arylamino)thiocarbonyl, Arylalkylaminocarbonyl, Arylsulfonyl, Arylalkyl, Arylalkenyl, Arylalkinyl, Arylalkylcarbonyl, Arylalkenylcarbonyl, Arylalkoxycarbonyl, Aryl(alkylthio)carbonyl, wobei der Alkylrest jeweils 1 bis 5 C-Atome enthalten kann und R⁶ wie oben definiert ist
oder mit bis zu drei voneinander unabhängigen Resten R⁶ substituiertes Heteroaryl, Heteroarylalkyl, Heteroarylalkenyl, Heteroarylalkylcarbonyl oder Heteroarylalkenylcarbonyl, Heteroaryloxycarbonyl, (Heteroarylthio)-carbonyl, Heteroarylaminocarbonyl, Heteroarylalkyloxycarbonyl, Hetero-aryl(alkylthio)carbonyl, Heteroarylalkylaminocarbonyl, wobei der Alkylrest jeweils 1 bis 3 C-Atome enthalten kann,
R³ und R⁴ gleich oder verschieden, unabhängig voneinander Wasserstoff, C₁-C₈-Alkyl, gegebenenfalls substituiert mit Fluor, Chlor, Hydroxy, Amino, Mercapto, C1 - C4-Acyloxy, Benzoyloxy, Benzyloxy, Phenoxy, C₁-C₄-Alkoxy, C₁-C₄-Alkylamino, Di-(C₁-C₄-alkyl)amino, C₁-C₄-Alkylthio, C₁-C₄-Alkylsulfonyl, C₁-C₄-Alkylsulfinyl, Carboxy, Carbamoyl;
C₂-C₈-Alkenyl, gegebenenfalls substituiert mit Fluor oder Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₃-C₈-Cycloalkyl, gegebenenfalls substituiert mit Fluor, Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₃-C₈-Cycloalkenyl, gegebenenfalls substituiert mit Fluor oder Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
mit bis zu fünf voneinander unabhängigen Resten R⁶ substituiertes Aryl, Arylalkyl, Heteroaryl oder Heteroarylalkyl bedeuten, wobei der Alkylrest jeweils 1 bis 3 C-Atome enthalten kann und R⁶ wie oben definiert ist,
R³ und R⁴ oder R³ und R⁵ können ferner auch
Teil eines gesättigten oder ungesättigten carbo- oder heterocyclischen Ringes mit 3 bis 8 C-Atomen sein, der gegebenenfalls mit Fluor, Chlor, Hydroxy, Amino, C₁-C₆-Alkyl, C₂-C₆-Alkenyl, C₂-C₆-Alkinyl, C₁-C₆-Acyloxy, Benzoyloxy, C₁-C₆-Alkoxy, Oxo, Thioxo, Carboxy, Carbamoyl oder Phenyl substituiert sein kann,
X bedeutet Sauerstoff, Schwefel, Selen oder substituierten Stickstoff N-R², worin R² die oben gegebenen Bedeutungen haben kann,
mit Ausnahme der Verbindungen, in denen R³ und R⁴ gleichzeitig H bedeuten und Verbindungen, in denen R² und R⁵ H bedeuten und R³ und/oder R⁴ Arylalkyl bedeuten und Verbindungen, in denen X Sauerstoff und R² und R⁵ Wasserstoff bedeuten.

2. Arzneimittel gemäß Anspruch 1, enthaltend eins oder mehrere der Chinoxaline der allgemeinen Formel (I) und (Ia),
in welchen
2)
n null,
eins,
zwei
oder drei,
die einzelnen Substituenten R¹ unabhängig voneinander
Fluor, Chlor, Brom, Trifluormethyl, Trifluormethoxy, Hydroxy, C₁-C₄-Alkyl, C₅-C₆-Cycloalkyl, C₁-C₄-Alkoxy, (C₁-C₄-Alkoxy)-(C₁-C₄-alkoxy), C₁-C₄-Alkylthio, C₁-C₄-Alkyl-sulfinyl, C₁-C₄-Alkylsulfonyl, Nitro, Amino, C₁-C₄-Alkylamino, Di-(C₁-C₄-alkyl)amino, Piperidino, Morpholino, 1-Pyrrolidinyl, 4-Methyl-piperazinyl, Thiomorpholino, Imidazolyl, C₁-C₄-Acyl, C₁-C₄-Acyloxy, C₁-C₄-Acylamino, Cyano, Carbamoyl, Carboxy, (C₁-C₄-Alkyl)oxycarbonyl, Hydroxysulfonyl, Sulfamoyl
oder
einen mit bis zu zwei
voneinander unabhängigen Resten R⁶ substituierten Phenyl-, Phenoxy-, Phenoxycarbonyl, Phenylthio-, Phenylsulfinyl, Phenylsulfonyl-, Phenoxy-sulfonyl-, Phenylsulfonyloxy-, Anilinosulfonyl, Phenylsulfonylamino, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C₁-C₄-Alkyl, C₃-C₇-Cycloalkyl, C₁-C₄-Alkoxy, C₁-C₄-Alkylthio, C₁-C₄-Alkyl-sulfinyl, C₁-C₄-Alkylsulfonyl, C₁-C₄-Alkylamino, Di(C₁-C₄-alkyl)-amino, (C₁-C₄-Alkyl)-oxycarbonyl, Phenyl, Phenoxy
sein kann,
R² Wasserstoff und R⁵
Wasserstoff, Hydroxy, Cyano, Amino,
C₁-C₆-Alkyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₂-C₈-Alkenyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₃-C₈-Allenyl,
C₃-C₈-Alkinyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₃-C₈-Cycloalkyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₃-C₈-Cycloalkenyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
(C₃-C₈-Cycloalkyl)-(C₁-C₂-alkyl),
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
(C₃-C₈-Cycloalkenyl)-(C₁-C₂-alkyl),
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₁-C₆-Alkylcarbonyl,
gegebenenfalls substituiert mit
Fluor, Chlor, Brom, Jod, Cyano, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, Oxo, Thioxo, Carboxy, Carbamoyl;
C₂-C₆-Alkenylcarbonyl, gegebenenfalls substituiert durch Fluor, Chlor oder Hydroxy, C₁-C₄-alkoxy, Oxo, Phenyl;
(C₃-C₆-Cycloalkyl)carbonyl, gegebenenfalls substituiert durch Fluor, Chlor oder Hydroxy, C₁-C₄-alkoxy, Oxo, Phenyl;
(C₅-C₆-Cycloalkenyl)carbonyl, gegebenenfalls substituiert durch Fluor, Chlor oder Hydroxy, C₁-C₄-alkoxy, Oxo, Phenyl;
(C₃-C₆-Cycloalkyl)-(C₁-C₂-alkyl)carbonyl, gegebenenfalls substituiert durch Fluor, Chlor oder Hydroxy, C₁-C₄-alkoxy, Oxo, Phenyl;
(C₅-C₆-Cycloalkenyl)-(C₁-C₂-alkyl)carbonyl, gegebenenfalls substituiert durch Fluor, Chlor oder Hydroxy, C₁-C₄-alkoxy, Oxo, Phenyl;
C₁-C₆-Alkyloxycarbonyl, gegebenenfalls substituiert durch Fluor, Chlor, Brom, Hydroxy, C₁-C₄-Alkoxy, C₁-C₄-Alkylamino, Di(C₁-C₄-alkyl)amino, C₁-C₄-Alkylthio;
C₂-C₆-Alkenyloxycarbonyl, gegebenenfalls substituiert durch Fluor, Chlor, Hydroxy, C₁-C₄-Alkoxy, Oxo, Phenyl;
C₂-C₆-Alkinyloxycarbonyl, gegebenenfalls substituiert durch Fluor, Chlor, Hydroxy, C₁-C₄-Alkoxy, Oxo, Phenyl;
C₁-C₆-Alkylthiocarbonyl,gegebenenfalls substituiert durch Fluor, Chlor, Hydroxy, C₁-C₄-Alkoxy, Oxo, Phenyl;
C₂-C₆-Alkenylthiocarbonyl, gegebenenfalls substituiert durch Fluor, Chlor, Hydroxy, C₁-C₄-Alkoxy, Oxo, Phenyl;
C₁-C₆-Alkylamino- und Di(C₁-C₆-alkyl)aminocarbonyl, gegebenenfalls substituiert durch Fluor, Chlor, Hydroxy, C₁-C₄-Alkoxy, Oxo, Phenyl;
Pyrrolidin-1-yl, Morpholino-, Piperidino-, Piperazinyl-, oder 4-Methylpiperazin-1-yl-carbonyl;
C₂-C₆-Alkenylamino- und Di(C₁-C₆-alkenyl)aminocarbonyl, gegebenenfalls substituiert durch Fluor, Chlor, Hydroxy, C₁-C₄-Alkoxy, Oxo, Phenyl;
C₁-C₄-Alkylsulfonyl, gegebenenfalls substituiert durch Fluor, Chlor, Hydroxy, C₁-C₄-Alkoxy, Oxo, Phenyl;
C₁-C₄-Alkenylsulfonyl, gegebenenfalls substituiert durch Fluor, Chlor, Hydroxy, C₁-C₄-Alkoxy, Oxo, Phenyl;
oder mit bis zu drei voneinander unabhängigen Resten R⁶ substituiertes
Aryl, Arylcarbonyl, Aryl(thiocarbonyl), (Arylthio)carbonyl, (Arylthio)thiocarbonyl, Aryloxycarbonyl, Arylaminocarbonyl, (Arylamino)thiocarbonyl, Arylalkylaminocarbonyl, Arylsulfonyl, Arylalkyl, Arylalkenyl, Arylalkinyl, Arylalkylcarbonyl, Arylalkenylcarbonyl, Aryl(alkylthio)carbonyl, Arylalkoxycarbonyl, wobei der Alkylrest jeweils 1 bis 5 C-Atome enthalten kann und R⁶ wie oben definiert ist
oder mit bis zu zwei voneinander unabhängigen Resten R⁶ substituiertes
1- oder 2-Naphthylmethyl, 2-, 3- oder 4-Picolyl, 2- oder 3-Furylmethyl, 2- oder 3-Thienylmethyl, 2- oder 3-Pyrrolylmethyl, 2-, 3- oder 4-Pyridylcarbonyl, 2- oder 3-Furylcarbonyl, 2- oder 3-Thienylcarbonyl, 2- oder 3-Thienylacetyl, 2-, 3- oder 4-Picolyloxycarbonyl, 2-, oder 3-Furylmethyloxycarbonyl, 2- oder 3-Thienylmethyloxycarbonyl,
und
R³ und R⁴ gleich oder verschieden, unabhängig voneinander
Wasserstoff,
C₁-C₆-Alkyl,
gegebenenfalls substituiert mit Fluor, Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₂-C₈-Alkenyl, gegebenenfalls substituiert mit Fluor oder Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₃-C₈-Cycloalkyl, gegebenenfalls substituiert mit Fluor, Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
C₃-C₈-Cycloalkenyl, gegebenenfalls substituiert mit Fluor oder Chlor, Hydroxy, Amino, Mercapto, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio, C₁-C₄-Alkylsulfonyl, C₁-C₄-Alkylsulfinyl, Carboxy, Carbamoyl;
mit bis zu drei voneinander unabhängigen Resten R⁶ substituiertes Aryl, Arylalkyl, Heteroaryl oder Heteroarylalkyl bedeuten, wobei der Alkylrest jeweils 1 bis 3 C-Atome enthalten kann und R⁶ wie oben definiert ist,
R³ und R⁴ können ferner auch
Teil eines gesättigten oder ungesättigten carbo- oder heterocyclischen Ringes mit 3 bis 7 C-Atomen sein, der gegebenenfalls mit Fluor, Chlor, Hydroxy, Amino, C₁-C₄-Alkyl, C₂-C₄-Alkenyl, C₂-C₄-Alkinyl, C₁-C₄-Acyloxy, Benzoyloxy, C₁-C₄-Alkoxy, Oxo, Thioxo, Carboxy, Carbamoyl oder Phenyl substituiert sein kann,
X bedeutet Sauerstoff, Schwefel oder Selen
gegebenenfalls in einer isomeren Form.

3. Arzneimittel gemäß Anspruch 1, enthaltend eins oder mehrere Chinoxaline der allgemeinen Formeln (I) und (Ia),
in welcher
n null,
eins
oder zwei,
die einzelnen Substituenten R¹ unabhängig voneinander
Fluor, Chlor, Brom, Trifluormethyl, Hydroxy, C₁-C₄-Alkyl, C₁-C₄-Alkoxy, (C₁-C₄-Alkoxy)-(C₁-C₂-alkoxy), C₁-C₄-Alkylthio, Nitro, Amino, C₁-C₄-Alkylamino, Di(C₁-C₄-alkyl)amino, Piperidino, Morpholino, 1-Pyrrolidinyl, 4-Methylpiperazinyl, C₁-C₄-Acyl, C₁-C₄-Acyloxy, C₁-C₄-Acylamino, Cyano, Carbamoyl, Carboxy, (C₁-C₄-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C₁-C₄-Alkyl, C₁-C₄-Alkoxy, (C1 - C4-Alkyl)-oxycarbonyl, Phenyl, Phenoxy
sein kann,
R² Wasserstoff und R⁵
C₁-C₆-Alkyl
gegebenenfalls substituiert mit C₁-C₄-Alkoxy oder C₁-C₄-Alkylthio;
C₂-C₆-Alkenyl,
gegebenenfalls substituiert mit Oxo;
C₃-C₆-Allenyl;
C₃-C₈-Alkinyl, insbesondere 2-Butinyl;
C₃-C₆-Cycloalkyl;
C₅-C₆-Cycloalkenyl;
(C₃-C₆-Cycloalkyl)-(C₁-C₂-alkyl), insbesondere Cyclopropylmethyl, gegebenenfalls substituiert mit C₁-C₄-Alkyl;
(C₃-C₆-Cycloalkenyl)-(C₁-C₂-alkyl), insbesondere Cyclohexenylmethyl;
C₁-C₆-Alkylcarbonyl,
gegebenenfalls substituiert mit Fluor,
Chlor, Hydroxy, Benzyloxy, Phenoxy, C₁-C₄-Alkoxy, C₁-C₄-Alkylamino, C₁-C₄-Alkenylamino, Di(C₁-C₄-alkyl)amino, 1-Pyrrolidinyl, Piperidino, Morpholino, 4-Methylpiperazin-1-yl, C₁-C₄-Alkylthio;
C₂-C₆-Alkenylcarbonyl;
C₁-C₆-Alkyloxycarbonyl, gegebenenfalls substituiert durch Fluor, Chlor, Brom, Hydroxy, C₁-C₄-Alkoxy, C₁-C₄-Alkylamino, Di(C₁-C₄-alkyl)amino, C₁-C₄-Alkylthio;
C₂-C₆-Alkenyloxycarbonyl, insbesondere Vinyloxycarbonyl, Allyloxycarbonyl, Isopropenyloxycarbonyl, Butenyloxycarbonyl, Pentenyloxycarbonyl;
C₂-C₆-Alkinyloxycarbonyl, insbesondere Propinyloxycarbonyl, Butinyloxycarbonyl;
C₁-C₆-Alkylthiocarbonyl;
C₂-C₆-Alkenylthiocarbonyl, insbesondere Allylthiocarbonyl;
C₁-C₆-Alkylamino- und Di(C₁-C₆-alkyl)aminocarbonyl;
Pyrrolidin-1-yl, Morpholino-, Piperidino-, Piperazinyl-, oder 4-Methylpiperazin-1-yl-carbonyl;
C₂-C₆-Alkenylamino- und Di(C₁-C₆-alkenyl)aminocarbonyl;
C₁-C₄-Alkylsulfonyl;
C₁-C₄-Alkenylsulfonyl;
oder mit bis zu zwei voneinander unabhängigen Resten R⁶ substituiertes Aryl, insbesondere Phenyl, Arylcarbonyl, insbesondere Benzoyl, (Arylthio)carbonyl, Aryloxycarbonyl, Arylaminocarbonyl, (Arylamino)thiocarbonyl, Arylalkylaminocarbonyl, Arylsulfonyl, Arylalkyl, insbesondere Benzyl, Phenylethyl, Arylalkenyl, Arylalkylcarbonyl, Arylalkoxycarbonyl, Aryl(alkylthio)carbonyl, wobei der Alkylrest jeweils 1 bis 3 C-Atome enthalten kann und R⁶ wie oben definiert ist
oder mit bis zu zwei voneinander unabhängigen Resten R⁶ substituiertes 1- oder 2-Naphthylmethyl, 2-, 3- oder 4-Picolyl, 2- oder 3-Furylmethyl, 2- oder 3-Thienylmethyl, 2- oder 3-Pyrrolylmethyl, 2-, 3- oder 4-Pyridylcarbonyl, 2- oder 3-Furylcarbonyl, 2- oder 3-Thienylcarbonyl, 2- oder 3-Thienylacetyl, 2-, 3- oder 4-Picolyloxycarbonyl, 2- oder 3-Furylmethyloxycarbonyl, 2- oder 3-Thienylmethyloxycaronyl,
und
R³ und R⁴ gleich oder verschieden, unabhängig voneinander
Wasserstoff,
C₁-C₄-Alkyl,
gegebenenfalls substituiert mit Hydroxy, Mercapto, C₁-C₄-Alkoxy, C₁-C₄-Alkylthio, C₁-C₄-Alkylsulfonyl, C₁-C₄-Alkylsulfinyl, Carboxy, Carbamoyl;
C₂-C₆-Alkenyl,
mit bis zu zwei voneinander unabhängigen Resten R⁶ substituiertes Aryl, Benzyl, Thienyl oder Thienylmethyl, wobei R⁶ wie oben definiert ist, bedeuten,
R³ und R⁴ können auch
Teil eines gesättigten oder ungesättigten carbo- oder heterocyclischen Ringes mit 3 bis 6 C-Atomen sein, der gegebenenfalls mit Oxo oder Thioxo substituiert sein kann und
X bedeutet Sauerstoff oder Schwefel
gegebenenfalls in einer isomeren Form.

4. Arzneimittel gemäß den Ansprüchen 1 bis 3, enthaltend als Proteaseinhibitor eine oder mehrere Verbindungen aus der Gruppe
1. (S)-N-[(alphaS)-alpha-[(1R)-2-[((3S,4aS,8aS)-3-(tert.butylcarbamoyl)octa-hydro-2(1H)-isoquinolinyl)-1-hydroxyethyl)phenethyl-2-quinaldamido]-succinamid
2. 2(R)-Benzyl-5-(2(S)-(N-tert.butylcarbamoyl)-4-(3-pyridylmethyl)piperazin-1-yl)-4(S)-hydroxy-N(2(R)-hydroxyindem-1(S)-yl)pentanamide
3. N-(Quinolin-2-ylcarbonyl)-asparagine-1(S)-benzyl-3-(3-tert.butyl-1-isobutylureido)-2(R)-hydroxypropylamide
4. N1-(2R-hydroxy-3-((3-methylbutyl)methylsulfonyl)amino)-1S-(phenyl-methyl)propyl)-2S-((2-quinolinylcarbonyl)amino)butanediamid
5. (2S,3S,5S)-5(N-(N-((N-methyl-N-((2-isopropyl-4-oxazolyl)methyl)amino)-carbonyl)valinyl)amino)-2-(N-((5-thiazolyl)methoxycarbonyl)amino)-1,6-diphenyl-3-hydroxyhexane
6. (R)-N-tert.butyl-3-((2S,3S)-2-hydroxy-3-N-((R)-2-N-(isoquinolin-5-yloxy-acetyl)amino-3-methylthiopropanoyl)amino-4-phenylbutanoyl)-5,5-dimethyl-1,3-thiazolidine-4-carboxamide
7. {3-[(4-Amino-benzenesulfonyl)-isobutyl-amino]-1-benzyl-2-hydroxypropyl}-carbamic acid tetrahydro-furan-3-yl-ester
8. (3S,6R)-3-(α-ethylbenzyl)-6-(α-ethylphenethyl)-4-hydroxy-2H-pyran-2-one (VB 11 478, PCT WO 9411361)
9. N-[5-L-[N-(2-quinolinecarbonyl)-L-asparaginyl]amino-(4R,3S)-epoxy-6-phenyl-hexanoyl]-isoleucin
10. N-tert.butyl-1-[2-(R)-hydroxy-4-phenyl)-3(S)-[[N-(2-quinolinylcarbonyl)-asparaginyl]amino]butyl-4(R)-(phenylthio)piperidin-2(S)-carboxamide
11. [3'''S-(3'''R*,4'''S*)]-N[1'-oxo-1'-(3''-[1'''-oxo-2'''-aza-3'''-phenylmethyl-4'''-hydroxy-5'''-(2'''-N-tert.butylcarbamido)phenyl]pentyl-4''-methyl)-1,2,3,4-tetrahydroisoquinoline
12. 2-[2-Hydroxy-3-(3-hydroxy-2-methyl-benzoylamino)-4-phenylsulfanyl-butyl]-decahydro-isoquinoline-3-carboxylicacid-tert.butylamide
13. 2H-1,4-Diazepin-2-one,hexahydro-6-hydroxy-1,3,4,7-tetrakis(phenylmethyl)-, [3S-(3.alpha., 6.beta., 7.beta)].

5. Arzneimittel, enthaltend in Kombination S-4-Isopropoxycarbonyl-6-methoxy-3-(methylthio-methyl)-3,4-dihydro-chinoxazolin-2(1H)-thion der Formel (A) 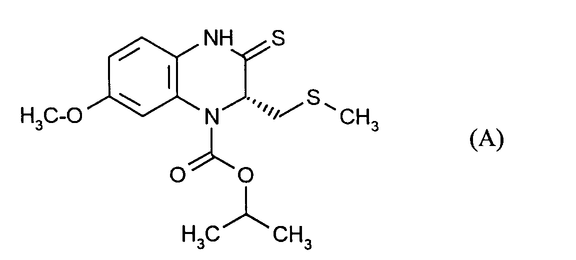 und (S)-N-[(alphaS)-alpha-[(1R)-2-[((3S,4aS,8aS)-3-(tert.butylcarbamoyl)octa-hydro-2(1H)-isoquinolinyl)-1-hydroxyethyl)phenethyl-2-quinaldamido]succinamid (Saquinavir) der Formel (B)
